# EUROPEAN PATENT APPLICATION

(11) **EP 4 825 429 A2**
(43) Date of publication of application: **21.10.2026**
(21) Application number: 26202952.3
(22) Date of filing: 11.03.2025
(51) Int. Cl.: A61M 25/10

(54) **BALLOON CATHETER WITH RETRACTABLE INNER SUPPORT**

(30) Priority: 21.10.2024 KR 20240143849 U
(62) Document number(s) of the earlier application(s) in accordance with Art. 76 EPC: 25162971.3 / 4 729 103
(71) Applicant: RADEXEL INC., Chuncheon-si, Gangwon-do 24437 (KR)
(72) Inventor: JUNG, Nuri Hyun, 24363 Chuncheon-si (KR); BAHNG, Jungbae, 30147 Sejong-si (KR); KIM, Seongjun, 04759 Seoul (KR); KIM, Tae Hoon, 03469 Seoul (KR)
(74) Representative: HGF

(57) **Abstract**

The present disclosure relates to a balloon catheter including an external catheter; a balloon connected to one side of the external catheter and capable of being inflated or contracted and inserted into a target site of a living body; an internal catheter having one side inserted into the balloon and the other side penetrating into an inside of the external catheter and capable of moving in a first direction inserted into the balloon or a second direction discharged from the balloon; and a driving part configured to move and discharge the internal catheter inserted into the inside of the balloon in the second direction in a state that the balloon is inflated and fixed to the target site of the living body.

## Description

### [TECHNICAL FIELD]

The present disclosure relates to a balloon catheter.

### [BACKGROUND ART]

Radiation therapy is to irradiate a lesion of a living body with radiation such as X-rays, gamma rays, electron rays, and proton rays to delay or destroy the growth of the lesion tissue of the living body. Here, the lesion tissue may be cancer, and the like.

During radiation therapy, the distribution of the radiation dose may change depending on the density of the medium located in the path through which the radiation beam penetrates the living body. Specifically, if two media with different densities exist in the path through which the radiation penetrates the living body, the distribution of the radiation dose in the boundary area of the two media may be greater than in other areas.

During radiation therapy, to reduce the radiation effect on the surrounding tissue of the lesion tissue of the living body, a balloon may be inserted into the body cavity (hereinafter referred to as the "target site") around the lesion tissue of the living body to fix the lesion tissue and the target site of the living body. Here, the balloon may be inserted into the target site of the living body through an internal catheter (or guide wire). Specifically, for example, the internal catheter may be inserted into the inside of the balloon, and then the balloon and the internal catheter may be inserted into the target site of the living body. At this time, the internal catheter inside the balloon caused the following problems.

First, the internal catheter inside the balloon made it impossible to lower the internal density of the balloon below a certain level. For reference, increasing the density inside the balloon is easy to achieve by simply injecting a high-density material into the balloon.

In addition, the internal catheter inside the balloon causes interference with particle beams during radiation therapy using particles such as electrons, protons, and carbon ions, which changes the point of arrival of the particle beams.

Furthermore, the internal catheter inside the balloon changes its position inside the balloon, causing uncertainty in the radiation dose distribution inside and around the balloon.

In addition, the internal catheter inside the balloon causes secondary electrons to be generated inside the balloon when performing MCRT (Magnetic Controlled Radiation Therapy) that controls the path of radiation using a magnetic field during radiation therapy such as X-rays, reducing the effect of radiation dose control.

Therefore, it is necessary to eject the internal catheter inside the balloon while the balloon is inserted into the target area of the living body. In this case, the general method of ejecting the internal catheter is to eject the internal catheter from inside the balloon while the balloon is not inflated. However, this method has the following problems.

First, there is a problem that the position of the balloon changes due to various directional forces from the internal organs and muscles inside the living body at the moment the internal catheter is ejected from inside the balloon while the balloon is inserted into the target area of the living body.

In addition, when there is pressure inside the living body, there is a problem that the balloon get stuck in the internal catheter at the moment the internal catheter is ejected from inside the balloon while the balloon is inserted into the target area of the living body.

It is important for radiation therapy to maintain the position of the balloon at the target area of the living body. Therefore, the conventional method of ejecting the internal catheter from inside the balloon while the balloon is not inflated is difficult to apply due to changes in the position of the balloon, and as a result, the balloon capable of ejecting the internal catheter has not been used during radiation therapy.

### [Prior art document]

(Patent Document 1) Korea Patent Registration No. 10-2013-0009445 (Published on January 23, 2013)

### [DETAILED DESCRIPTION OF THE INVENTION]

### [TECHINICAL PROBLEM]

The present disclosure is to provide a balloon catheter that can improve the accuracy of radiation therapy for lesion tissue and target area of a living body by allowing an internal catheter inserted into the balloon to be discharged while the balloon is inflated and fixes the lesion tissue and target area of the living body.

The problems to be solved by the present disclosure are not limited to the problems mentioned above, and other problems that are not mentioned will be clearly understood by those skilled in the art from the description below.

### [TECHNICAL SOLUTION]

According to an embodiment of the present disclosure, a balloon catheter includes an external catheter; a balloon connected to one side of the external catheter and capable of being inflated or contracted and inserted into a target site of a living body; an internal catheter having one side inserted into the balloon and the other side penetrating into an inside of the external catheter and capable of moving in a first direction inserted into the balloon or a second direction discharged from the balloon; and a driving part configured to move and discharge the internal catheter inserted into the inside of the balloon in the second direction in a state that the balloon is inflated and fixed to the target site of the living body.

Furthermore, the external catheter may include a coupling part coupled to an outer circumference of the internal catheter; a stretchable part coupled to the coupling part and capable of being stretched; and a guide part coupled to the stretchable part and configured to guide a movement of the internal catheter, and wherein the balloon may be connected to the guide part.

Furthermore, the stretchable part may have a bellows shape.

Furthermore, the balloon catheter may further include a first stopper configured to limit a movement range of the internal catheter in the second direction.

Furthermore, the first stopper may include a hook groove formed in the external catheter; and a hook formed in the internal catheter and hooked to the hook groove when the internal catheter moves in the second direction.

Furthermore, the balloon catheter may further include a second stopper configured to limit a movement range of the internal catheter in the first direction and a movement range of the second direction.

Furthermore, the second stopper may include two catch protrusions spaced apart with a spacing and connected along a movement direction of the internal catheter to the outer circumference of the external catheter; and a moving protrusion connected to the outer circumference of the internal catheter and is arranged between the two catch protrusions.

Furthermore, the driving part may include an actuator configured to move the internal catheter in the second direction.

Furthermore, the driving part may include a fluid supply part configured to inject fluid into the balloon so that the internal catheter moves in the second direction by the fluid injected into the balloon.

Furthermore, the balloon catheter may further include a third stopper detachably fixed to the internal catheter and configured to limit a movement range of the internal catheter in the first direction.

Furthermore, the third stopper may have a U-shape.

Other specific details of the present disclosure are included in the detailed description and drawings.

### [ADVANTAGEOUS EFFECTS OF THE INVENTION]

According to one embodiment of the present disclosure, the balloon catheter can discharge the internal catheter inserted inside the balloon while the balloon is inflated and fixes lesion tissue and target area of a living body, thereby improving the accuracy of radiation therapy for the lesion tissue and target area of the living body during radiation therapy.

The effects of the present disclosure are not limited to the effects mentioned above, and other effects not mentioned will be clearly understood by those skilled in the art from the description below.

### [DESCRIPTION OF THE DRAWINGS]

FIG. 1 is a cross-sectional view illustrating a balloon catheter according to one embodiment of the present disclosure.
FIG. 2 is a cross-sectional view illustrating the balloon of FIG. 1 in an inflated state.
FIG. 3 is a cross-sectional view illustrating a first stopper of a balloon catheter according to an embodiment of the present disclosure.
FIG. 4 is a cross-sectional view illustrating the balloon of FIG. 3 in an inflated state.
FIG. 5 is a cross-sectional view illustrating a second stopper of a balloon catheter according to one embodiment of the present disclosure.
FIG. 6 is a cross-sectional view illustrating the balloon of FIG. 5 in an inflated state.
FIGS. 7 to 10 are cross-sectional views illustrating an operation process of a balloon catheter according to another embodiment of the present disclosure.
FIG. 11 is a schematic diagram illustrating a state in which a balloon of a balloon catheter according to one embodiment of the present disclosure is fixed to a target site of a living body.
FIG. 12 is a schematic diagram illustrating a state in which a balloon of a conventional balloon catheter is fixed to a target site of a living body.
FIG. 13 is a cross-sectional diagram illustrating a process of radiation therapy of a target site of a living body using a balloon catheter according to one embodiment of the present disclosure.
FIG. 14 is a cross-sectional diagram illustrating a process of radiation therapy of a target site of a living body using a conventional balloon catheter.
FIG. 15 is a cross-sectional view illustrating an MCRT treatment process of a target area of a living body using a balloon catheter according to one embodiment of the present disclosure.
FIG. 16 is a cross-sectional view illustrating an MCRT treatment process of a target area of a living body using a balloon catheter according to one embodiment of the present disclosure.

### [DETAILED DESCRIPTION]

The advantages and features of the present disclosure, and methods for achieving them, will become clear with reference to the embodiments described in detail below along with the accompanying drawings. However, the present disclosure is not limited to the embodiments disclosed below and may be implemented in various different forms. The present embodiments are merely provided to ensure that the present disclosure is complete, and provided to fully convey the scope of the present disclosure to those skilled in the art to which the present disclosure pertains. The present disclosure is defined only by the scope of the claims.

The terminology used herein is for the purpose of describing embodiments and is not intended to limit the disclosure. As used herein, singular forms also include plural forms, unless specifically stated otherwise in the context. As used in the specification, "comprises" and/or "comprising" does not exclude the presence or addition of one or more other elements in addition to the mentioned elements. The same reference numerals refer to the same elements throughout the specification, and "and/or" includes each and every combination of one or more of the referenced elements. Although "first", "second", and the like are used to describe various components, these components are of course not limited by these terms. These terms are merely used to distinguish one component from another. Therefore, the first component mentioned below may also be the second component within the technical spirit of the present disclosure.

Unless otherwise defined, all terms (including technical and scientific terms) used in this specification may be used with meanings commonly understood by those skilled in the art to which this disclosure pertains. Additionally, terms defined in commonly used dictionaries are not to be interpreted ideally or excessively unless clearly specifically defined.

Hereinafter, the embodiments of the present disclosure will be described in detail with reference to the attached drawings.

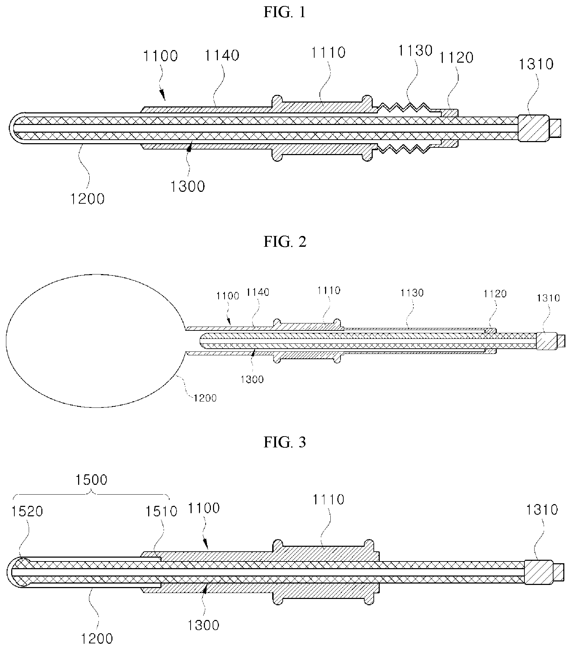

FIG. 1 is a cross-sectional view illustrating a balloon catheter according to one embodiment of the present disclosure, and FIG. 2 is a cross-sectional view illustrating the balloon of FIG. 1 in an inflated state.

As shown in FIG. 1, the balloon catheter according to one embodiment of the present disclosure may include an external catheter 100, a balloon 200, an internal catheter 300, and a driving part.

The external catheter 100 may serve as a basic body of the present disclosure. The external catheter 100 may be open on both sides. Here, one side of the external catheter 100 may be connected by the balloon 200. In addition, the interior of the external catheter 100 may be penetrated by the internal catheter 300. Furthermore, a handle 110 for grasping the external catheter 100 may be formed on the other side of the external catheter 100. Here, the center of the handle 110 may have a smaller outer diameter than the two sides of the handle 110, in other words, the two sides of the handle 110 may have a multi-stage shape that protrudes from the center of the handle 110. Accordingly, the two sides of the handle 110 may serve to prevent the operator's hand grasped at the center of the handle 110 from being separated in the longitudinal direction of the handle 110.

As an example, the outer diameter of the external catheter 100 may be 10 mm to 30 mm, but the present disclosure is not limited thereto. As another example, the length of the external catheter 100 may be 50 mm to 1,000 mm, but the present disclosure is not limited thereto. As another example, the material of the external catheter 100 may include at least one of silicone, latex, polyurethane, polyisoprene, and PVC.

The external catheter 100 may include a coupling part 120, a stretchable part 130, and a guide part 140. Here, the guide part 140 may be located on one side of the external catheter 100, the coupling part 120 may be located on the other side of the external catheter 100, and the stretchable part 130 may be located between the one side and the other side of the external catheter 100.

The coupling part 120 may be coupled to the outer circumference of the internal catheter 300. As an example, the coupling part 120 may have a ring shape.

The stretchable part 130 is connected to one side of the connecting part 120 and may be stretchable in the direction of movement of the internal catheter 300. For example, the stretchable part 130 may have a bellows shape.

The guide part 140 is connected to one side of the stretchable part 130 and may guide the movement of the internal catheter 300. For example, the guide part 140 may have a ring shape. Meanwhile, the balloon 200 may be connected to one side of the guide part 140.

The balloon 200 is connected to one side of the external catheter 100, may be inflated or contracted, and may be inserted into a target site of a living body. This balloon 200 may be inflated while inserted into the target site of the living body and fixed to a lesion tissue and the target site of the living body. For example, the balloon 200 may be inflated by fluid injection. For reference, the target site of the living body may be an internal space of the living body that may be inserted without an incision in the living body (e.g., one of the oral cavity, nasal cavity, pharynx, larynx, esophagus, stomach, duodenum, large intestine, and rectum), an internal space of the living body that may be inserted through an incision (e.g., one of the thoracic cavity, abdominal cavity, and inside the skin), but the present disclosure is not limited thereto, and may be applied to other tissues of the living body. In addition, the lesion tissue may be cancer tissue.

For example, the material of the balloon 200 may include at least one of silicone, latex, polyurethane, and polyisoprene.

The internal catheter 300 may have one side inserted into the inside of the balloon 200 and the other side penetrated into the inside of the external catheter 100, and may be moved in a first direction inserted into the balloon 200 or a second direction discharged from the balloon 200.

For example, when the internal catheter 300 is moved in the first direction inserted into the balloon 200, one side of the internal catheter 300 may be inserted into the inside of the balloon 200. At this time, the one side of the internal catheter 300 inserted into the inside of the balloon 200 may provide rigidity to the balloon 200 when the balloon 200 is inserted into the target site of the living body, thereby overcoming the insertion resistance applied to the balloon 200 in the living body. Thereafter, the balloon 200 inserted into the target site of the living body may be inflated and fixed to the lesion tissue and target site of the living body. Subsequently, one side of the internal catheter 300 inserted into the inside of the balloon 200 may be discharged from the inside of the balloon 200 by the driving part.

As an example, a valve 310 for sealing the internal catheter may be installed on the other side of the internal catheter 300. This valve 310 may serve to allow the inflow of the fluid supplied from a fluid supply part and prevent the outflow of the corresponding fluid.

As an example, the valve 310 may be composed of at least one of a check valve, a stopcock, and a clamp.

For example, the external catheter 100 or the internal catheter 300 may be fixed around the target site of the living body so that the position of the balloon 200 in the target site of the living body is maintained.

The driving part may move the internal catheter 300 inserted inside the balloon 200 in the second direction and discharge the internal catheter 300 while the balloon 200 inserted in the target site of the living body is inflated and fixed. This driving part may be operated under the control of a processor.

The driving part may include an actuator and the fluid supply part.

The actuator may move the internal catheter 300 in the second direction. For example, the actuator may move the internal catheter 300 in the second direction while the balloon 200 inserted in the target site of the living body is inflated and fixed under the control of the processor.

The fluid supply part may inject fluid into the balloon 200 so that the internal catheter 300 moves in the second direction by the fluid injected into the balloon 200. Here, the diameter and material of the balloon 200 may be initially set so that the pressure required for initial inflation is greater than the pressure required for the internal catheter 300 to move in the second direction. Therefore, in the case that the fluid supply part injects fluid into the interior of the balloon 200, the gas filled in the interior of the balloon 200 may push the internal catheter 300 so that the internal catheter 300 moves in the second direction.

The fluid supply part may inject fluid through the internal catheter 300 or the external catheter 100.

Hereinafter, a process of inserting and fixing a balloon catheter according to one embodiment of the present disclosure into the target site of the living body will be described.

First, the internal catheter 300 penetrated into the external catheter 100 is moved in the first direction and inserted into the inside of a balloon 200.

Subsequently, the balloon 200 is inserted into the target site of the living body. At this time, one side of the internal catheter 300 inserted into the inside of the balloon 200 may provide rigidity to the balloon 200 when the balloon 200 is inserted into the target site of the living body, thereby overcoming the insertion resistance applied to the balloon 200 inside the living body.

Next, the balloon 200 is inflated and fixed to the lesion tissue and target site of the living body. At this time, the balloon 200 may be inflated by fluid injection. For example, the fluid injection of the balloon 200 may be performed through the fluid supply part of the driving part.

Thereafter, the driving part moves the internal catheter 300 in the second direction so that one side of the internal catheter 300 is discharged from the inside of the balloon 200.

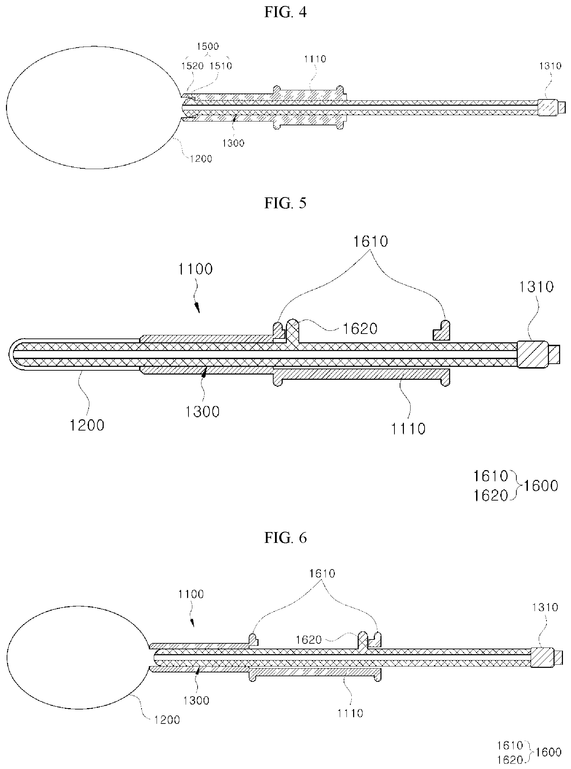

FIG. 3 is a cross-sectional view illustrating a first stopper of a balloon catheter according to an embodiment of the present disclosure, and FIG. 4 is a cross-sectional view illustrating the balloon of FIG. 3 in an inflated state.

As shown in FIG. 3, the balloon catheter according to an embodiment of the present disclosure may further include a first stopper 500, unlike the example of FIG. 1. In this example, the inner circumference of the external catheter 100 may be closely attached to the outer circumference of the internal catheter 300. Therefore, air leakage or fluid leakage between the external catheter 100 and the internal catheter 300 may be prevented.

The first stopper 500 may serve to limit the movement range of the internal catheter 300 in the second direction. The first stopper 500 may include a hook groove 510 and a hook 520.

The hook groove 510 may be formed on one side of the external catheter 100. For example, the hook groove 510 may be formed by being sunken along the inner circumference of one side of the external catheter 100. At this time, the hook groove 510 may have a ring shape.

The hook 520 is formed in the internal catheter 300 and may be caught in the hook groove 510 when the internal catheter 300 moves in the second direction. For example, the hook 520 may have a shape corresponding to the hook groove 510.

In this example, when the internal catheter 300 moves in the second direction, the hook 520 is caught in the hook groove 510 and the range of movement of the internal catheter 300 in the second direction may be limited (Refer to FIG. 4).

FIG. 5 is a cross-sectional view illustrating a second stopper of a balloon catheter according to one embodiment of the present disclosure, and FIG. 6 is a cross-sectional view illustrating the balloon of FIG. 5 in an inflated state.

As illustrated in FIG. 5, the balloon catheter according to one embodiment of the present disclosure may further include a second stopper 600, unlike the example of FIG. 1. In this example, the inner circumference of the external catheter 100 may be closely attached to the outer circumference of the internal catheter 300. Therefore, air leakage or fluid leakage through the space between the external catheter 100 and the internal catheter 300 may be prevented.

The second stopper 600 may limit the first direction movement range and the second direction movement range of the internal catheter 300. This second stopper 600 may include two catch protrusions 610 and a moving protrusion 620.

The two catch protrusions 610 may be coupled to the outer circumference of the external catheter 100 with a gap along the direction of movement of the internal catheter 300. For example, the two catch protrusions 610 may have a shape that protrudes vertically from the outer circumference of the external catheter 100.

The moving protrusion 620 may be coupled to the outer circumference of the internal catheter 300 and may be positioned between the two studs 610. For example, the moving protrusion 620 may have a shape that protrudes vertically from the outer circumference of the internal catheter 300.

In this example, when the internal catheter 300 moves in the first direction, the moving protrusion 620 may be caught by the one located closer to one side of the external catheter 100 among the two catch protrusions 610, and the movement range of the internal catheter 300 in the first direction may be limited (Refer to FIG. 6).

In addition, when the internal catheter 300 moves in the second direction, the moving protrusion 620 may be caught by the one located closer to the other side of the external catheter 100 among the two catch protrusions 610, and the movement range of the internal catheter 300 in the second direction may be limited (Refer to FIG. 6).

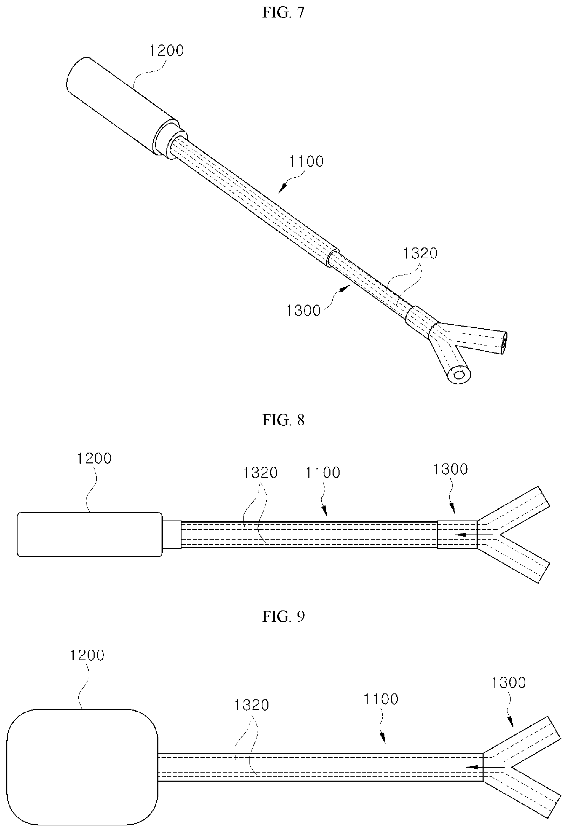

FIGS. 7 to 10 are cross-sectional views illustrating an operation process of a balloon catheter according to another embodiment of the present disclosure.

As shown in FIG. 7, the balloon catheter according to another embodiment of the present disclosure may have the internal catheter 300 with multiple lumens 320, unlike the example of FIG. 1, and may include a third stopper 700.

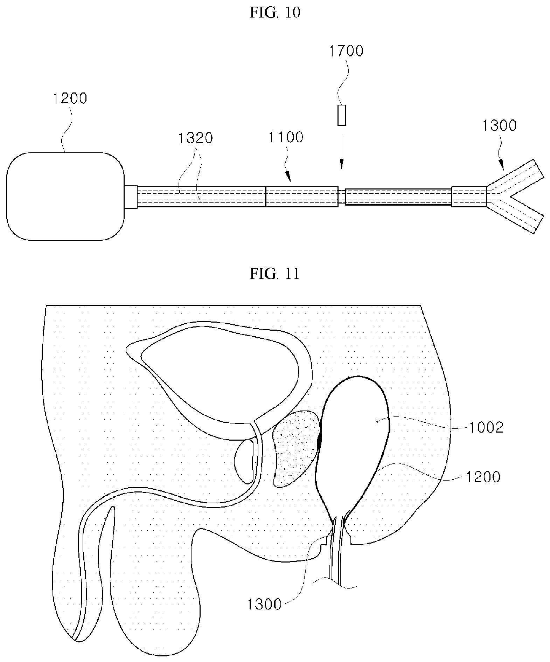

The third stopper 700 may be detachably fixed to the internal catheter 300 and may serve to limit the movement range of the internal catheter 300 in the first direction (Refer to FIG. 10). As an example, the third stopper 700 may have a U-shape. This third stopper 700 is fixed to the outer circumference of the internal catheter 300 in contact with the other side of the external catheter 100 while one side of the internal catheter 300 is discharged from the inside of the balloon 200, thereby limiting the range of movement of the internal catheter 300 in the first direction.

Hereinafter, a process of inserting and fixing the balloon catheter according to another embodiment of the present disclosure into a target site of a living body will be described.

First, the internal catheter 300 penetrated into the external catheter is moved in the first direction and inserted into the inside of the balloon 200 (Refer to FIG. 8).

Next, the balloon 200 is inserted into the target site of the living body. At this time, one side of the internal catheter 300 inserted into the inside of the balloon 200 may provide rigidity to the balloon 200 when the balloon 200 is inserted into the target site of the living body, thereby overcoming the insertion resistance applied to the balloon 200 inside the living body.

Thereafter, the balloon 200 is inflated and fixed to the lesion tissue and target site of the living body. At this time, the balloon 200 may be inflated by fluid injection. For example, the fluid injection of the balloon 200 may be performed through the fluid supply part of the driving part (Refer to FIG. 9).

Next, the driving part moves the internal catheter 300 in the second direction so that one side of the internal catheter 300 is discharged from the inside of the balloon 200. Thereafter, the third stopper 700 is fixed to the outer circumference of the internal catheter 300 that contacts the other side of the external catheter 100 while one side of the internal catheter 300 is discharged from the inside of the balloon 200, thereby limiting the movement range of the internal catheter 300 in the first direction (Refer to FIG. 10).

As an example, during radiation therapy, one of X-rays, electrons, protons, and carbon particles of 1 MeV or more may be used.

For reference, in the case that there is a balloon with a large or small density inside the living body compared to the target area of the living body, it may take more time to calculate the radiation dose for the radiation treatment plan. Therefore, the balloon 200 may be set to a predetermined shape and density to shorten the radiation dose calculation time. At this time, the shape of the balloon 200 may be set to one of a sphere, a hemisphere, an ellipsoid, a cylinder, and a square cylinder. Furthermore, the basic shape of the balloon 200 may be adjusted when it is ejected, and the expansion shape may be adjusted by adjusting the thickness of each unit area of the balloon 200. In addition, the internal density of the balloon 200 may be adjusted by adjusting the composition of the material injected into the balloon 200.

For example, a sensing module for measuring the volume and pressure of the balloon 200 may be installed in the external catheter 100 or the internal catheter 300, and the sensing module may provide the volume and pressure data of the balloon 200 to the operator for monitoring the volume and pressure of the balloon 200.

For example, the internal temperature of the balloon 200 may be controlled through a fluid circulation device that circulates the fluid injected into two or more lumens 320 of the internal catheter 300. At this time, the temperature of the balloon 200 may affect the therapeutic effect or side effect of the target area of the living body that comes into contact with the balloon 200, and the higher the temperature of the balloon 200, the greater the effect and side effect due to radiation, and the lower the temperature of the balloon 200, the less the effect and side effect due to radiation.

For example, the balloon 200 may be coated with a radiation-sensitive material that changes color when exposed to radiation. Accordingly, when the balloon 200 is exposed to radiation, the color of the balloon 200 may change, and through this, the radiation dose irradiated and absorbed by the balloon 200 may be calculated, and through this, the radiation dose irradiated and absorbed by the target area of the living body in contact with the balloon 200 may be calculated. Meanwhile, the color change of the balloon 200 may be identified in real time through an endoscope inserted into the target area of the living body, or may be identified through the balloon 200 discharged from the target area of the living body after the end of radiation treatment.

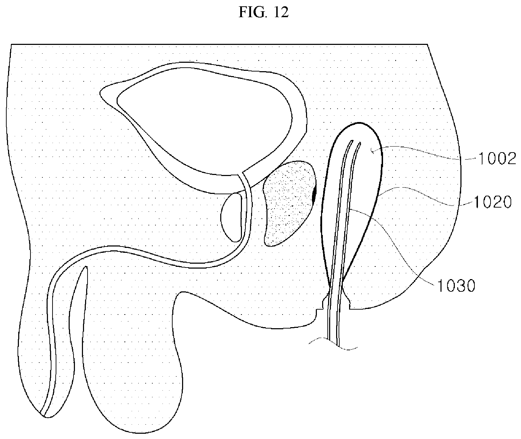

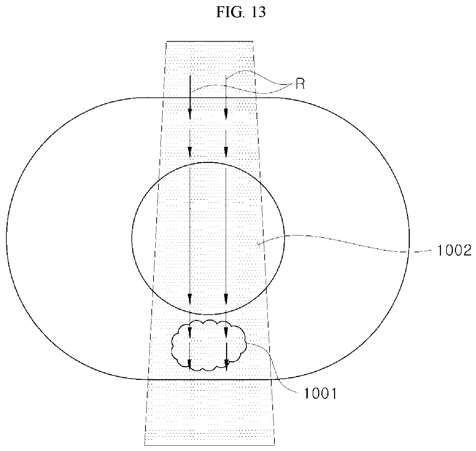

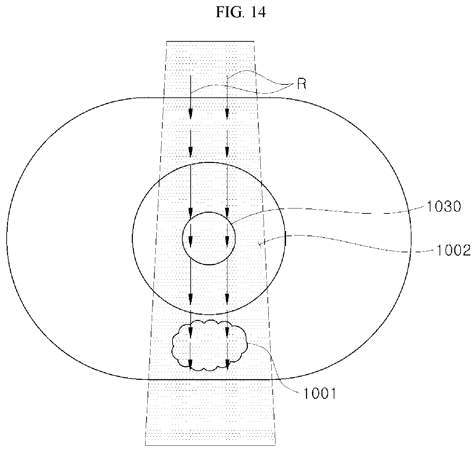

FIG. 11 is a schematic diagram illustrating a state in which a balloon of a balloon catheter according to one embodiment of the present disclosure is fixed to a target site of a living body, FIG. 12 is a schematic diagram illustrating a state in which a balloon of a conventional balloon catheter is fixed to a target site of a living body, FIG. 13 is a cross-sectional diagram illustrating a process of radiation therapy of a target site of a living body using a balloon catheter according to one embodiment of the present disclosure, and FIG. 14 is a cross-sectional diagram illustrating a process of radiation therapy of a target site of a living body using a conventional balloon catheter.

Referring to FIG. 11, in the state that the balloon 200 of the balloon catheter according to one embodiment of the present disclosure is fixed to a target site 1002 of the living body, the internal catheter 300 does not exist inside the balloon 200 fixed to the target site 1002 of the living body. Therefore, during radiation therapy, it may be identified that the radiation R penetrating the inside of the balloon 200 reaches the lesion tissue 1 of the living body without being affected by the internal catheter 300 (Refer to FIG. 13).

On the other hand, referring to FIG. 12, in the state that the balloon 1020 of the conventional balloon catheter is fixed to the target area of the living body, it may be identified that the internal catheter 1030 exists inside the balloon 1020 fixed to the target area 1002 of the living body. Therefore, during radiation therapy, it may be identified that the radiation R penetrating the inside of the balloon 200 is affected by the internal catheter 300 and reaches the lesion tissue 1 of the living body (Refer to FIG. 14). Due to this, the accuracy of radiation therapy in the conventional technology may be reduced.

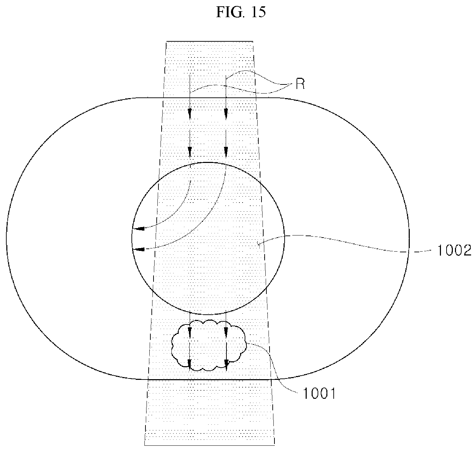

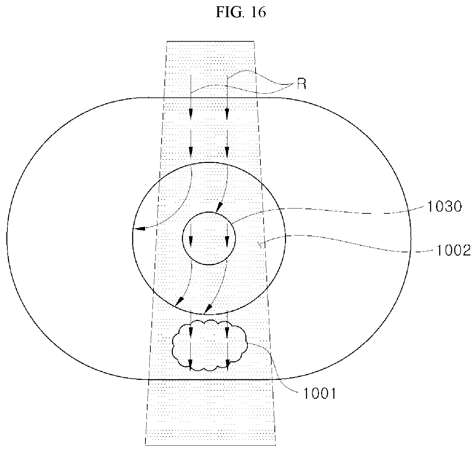

FIG. 15 is a cross-sectional view illustrating an MCRT treatment process of a target area of a living body using a balloon catheter according to one embodiment of the present disclosure, and FIG. 16 is a cross-sectional view illustrating an MCRT treatment process of a target area of a living body using a balloon catheter according to one embodiment of the present disclosure.

Referring to FIG. 15, in the balloon catheter according to one embodiment of the present disclosure, there is no internal catheter 300 inside the balloon 200 fixed to the target area 1002 of the living body. Accordingly, in MCRT (Magnetic Controlled Radiation Therapy) that controls the path of radiation using a magnetic field, since there is no internal catheter 300 on the path of radiation penetrating the inside of the balloon 200, the generation of secondary electrons due to the influence of the internal catheter 300 on the radiation may be prevented.

On the other hand, referring to FIG. 16, the conventional balloon catheter has an internal catheter 300 inside the balloon 200 fixed to the target site 1002 of the living body. Therefore, during MCRT, which controls the path of radiation using a magnetic field, since the internal catheter 1030 is on the path of radiation penetrating the inside of the balloon 1020, secondary electrons are generated due to the influence of the internal catheter 1030 on the radiation. Due to this, the conventional technology has a reduced accuracy in controlling the path of radiation by MCRT.

Therefore, the balloon catheter according to one embodiment of the present disclosure has the effect of improving the accuracy of radiation treatment for the lesion tissue and target site of the living body during radiation treatment by enabling the discharge of the internal catheter inserted inside the balloon while the balloon is inflated and fixing the lesion tissue and target site of the living body.

Although the embodiments of the present disclosure have been described above with reference to the attached drawings, those skilled in the art will understand that the present disclosure may be implemented in other specific forms without changing the technical idea or essential features thereof. Therefore, it should be understood that the embodiments described above are exemplary in all respects and not restrictive.

Further examples in accordance with the present disclosure are set out in the following numbered clauses.
1. A balloon catheter comprising:
   an external catheter;
   a balloon connected to one side of the external catheter and capable of being inflated or contracted and inserted into a target site of a living body;
   an internal catheter having one side inserted into the balloon and the other side penetrating into an inside of the external catheter and capable of moving in a first direction inserted into the balloon or a second direction discharged from the balloon; and
   a driving part configured to move and discharge the internal catheter inserted into the inside of the balloon in the second direction in a state that the balloon is inflated and fixed to the target site of the living body.
2. The balloon catheter of clause 1, wherein the external catheter comprises:
   a coupling part coupled to an outer circumference of the internal catheter;
   a stretchable part coupled to the coupling part and capable of being stretched; and
   a guide part coupled to the stretchable part and configured to guide a movement of the internal catheter, and wherein the balloon is connected to the guide part.
3. The balloon catheter of clause 2, wherein the stretchable part has a bellows shape.
4. The balloon catheter of clause 1, further comprising:
   a first stopper configured to limit a movement range of the internal catheter in the second direction.
5. The balloon catheter of clause 4, wherein the first stopper comprises:
   a hook groove formed in the external catheter; and a hook formed in the internal catheter and hooked to the hook groove when the internal catheter moves in the second direction.
6. The balloon catheter of clause 1, further comprising:
   a second stopper configured to limit a movement range of the internal catheter in the first direction and a movement range of the second direction.
7. The balloon catheter of clause 6, wherein the second stopper comprises:
   two catch protrusions spaced apart with a spacing and connected along a movement direction of the internal catheter to the outer circumference of the external catheter; and
   a moving protrusion connected to the outer circumference of the internal catheter and is arranged between the two catch protrusions.
8. The balloon catheter of clause 1, wherein the driving part comprises:
   an actuator configured to move the internal catheter in the second direction.
9. The balloon catheter of clause 1, wherein the driving part comprises:
   a fluid supply part configured to inject fluid into the balloon so that the internal catheter moves in the second direction by the fluid injected into the balloon.
10. The balloon catheter of clause 1, further comprising:
   a third stopper detachably fixed to the internal catheter and configured to limit a movement range of the internal catheter in the first direction.
11. The balloon catheter of clause 10, wherein the third stopper has a U-shape.

## Claims

1. A balloon catheter for use in radiation therapy, comprising:
an external catheter;
a balloon connected to one side of the external catheter, capable of being inflated or contracted, and configured to be inserted into a target site of a living body;
an internal catheter having one side inserted into the balloon and the other side penetrating into an inside of the external catheter, and capable of moving in a first direction to be inserted into the balloon and a second direction to be discharged from the balloon; and
a first stopper configured to limit a movement range of the internal catheter in the second direction,
wherein the internal catheter is configured to allow fluid to be supplied into the balloon through the internal catheter,
wherein an inner circumference of the external catheter and an outer circumference of the internal catheter are closely attached to each other so as to prevent air leakage or fluid leakage through a space between the external catheter and the internal catheter, and
wherein the one side of the internal catheter is configured to:
be positioned inside the balloon and provide rigidity to the balloon when the balloon is inserted into the target site of the living body; and
be movable in the second direction so as to be discharged from the inside of the balloon in a state in which the balloon is inflated by the fluid supplied through the internal catheter and fixed to the target site of the living body.

2. The balloon catheter of claim 1, further comprising:
a fluid supply part configured to supply the fluid into the balloon through the internal catheter.

3. The balloon catheter of claim 1,
wherein a valve configured to seal the internal catheter, allow inflow of fluid through the internal catheter, and prevent outflow of the fluid is provided on the other side of the internal catheter.

4. The balloon catheter of claim 3,
wherein the valve comprises at least one of a check valve, a stopcock, and a clamp.

5. The balloon catheter of claim 1,
wherein the first stopper comprises:
a hook groove formed in the external catheter; and
a hook formed in the internal catheter and configured to be hooked to the hook groove when the internal catheter moves in the second direction.

6. The balloon catheter of claim 1, further comprising:
a driving part configured to move the internal catheter in the second direction to discharge the one side of the internal catheter from the inside of the balloon in a state in which the balloon is inflated and fixed to the target site of the living body.

7. The balloon catheter of claim 6,
wherein the driving part comprises a fluid supply part configured to supply fluid into the balloon, and
wherein the fluid supply part is configured to supply the fluid into the balloon such that the internal catheter moves in the second direction by the fluid supplied into the balloon.
